# EUROPEAN PATENT APPLICATION

(11) **EP 2 275 138 A1**
(43) Date of publication of application: **19.01.2011**
(21) Application number: 09723897.6
(22) Date of filing: 24.03.2009
(51) Int. Cl.: A61K 45/00, A61K 31/137, A61K 31/198, A61K 38/00, A61P 1/04, A61P 1/14, C07K 5/083, C07K 5/093

(54) **PROMOTER FOR BICARBONATE SECRETION IN GASTROINTESTINAL TRACT**

(30) Priority: 24.03.2008 JP 2008076362
(71) Applicant: Ajinomoto Co., Inc., Tokyo, 104-8315 (JP)
(72) Inventor: TAKEUCHI, Koji, Kyoto-shi Kyoto 607-8414 (JP); AIHARA, Eitaro, Kyoto-shi Kyoto 607-8414 (JP); NAKAMURA, Eiji, Kawasaki-shi Kanagawa 210-8681 (JP); YANO, Tetsuo, Kawasaki-shi Kanagawa 210-8681 (JP); HASUMURA, Mai, Kawasaki-shi Kanagawa 210-8681 (JP); UNEYAMA, Hisayuki, Kawasaki-shi Kanagawa 210-8681 (JP)
(74) Representative: Strehl Schübel-Hopf & Partner
(86) International application number: PCT/JP2009/055769
(87) International publication number: WO 2009/119554

(57) **Abstract**

Disclosed is a substance which can prevent or treat a disease associated with the secretion of an acid. A calcium receptor activator is used as an active ingredient of a promoter for the bicarbonate secretion in a gastrointestinal tract. Examples of the calcium receptor activator include γ-Glu-X-Gly [wherein X represents an amino acid or an amino acid derivative], γ-Glu-Val-Y [wherein Y represents an amino acid or an amino acid derivative], γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH2, γ-Glu-Val-ol, γ-Glu-Ser, γ-Glu-Tau, γ-Glu-Cys(S-Me)(O), γ-Glu-Leu, γ-Glu-Ile, γ-Glu-t-Leu and γ-Glu-Cys(S-Me).

## Description

### Technical Field

The present invention relates to a bicarbonate secretion promoter in the gastrointestinal tract. The bicarbonate secretion promoter of the present invention can be utilized in a prophylactic or therapeutic agent for enhancing bicarbonate secretion in the prophylaxis or treatment of gastrointestinal dysfunctions such as acid secretion-related diseases, or a food effective for the prophylaxis or improvement of gastrointestinal dysfunctions.

### Background Art

Acid secretion-related diseases such as gastric ulcer, duodenal ulcer, non-steroidal anti-inflammatory drug (NSAID)-induced ulcer, gastro-esophageal reflux disease (GERM), and non-erosive reflux disease (NERD) are gastrointestinal diseases which are observed with high frequency in dairy clinical practice.

Peptic ulcers such as gastric ulcer and duodenal ulcer are diseases which form as a result of the exposure of the gastric and duodenal mucosa to an excess amount of acids or digestive enzymes, or which develop as a result of the breakdown of the mucosal defense mechanism. Peptic ulcers are considered to be caused by (1) infection with *Helicobacter pylori*, (2) disorders due to non-steroidal anti-inflammatory drugs and steroidal drugs, and (3) stress, drinking, and smoking, for example. Therapeutic drugs for the peptic ulcers are broadly classified into a medicament for inhibiting aggressive factors and a medicament for enhancing defensive factors. Examples of the medicament for inhibiting aggressive factors include a histamine H2 receptor inhibitor and a proton pump inhibitor each inhibiting acid secretion, and examples of the medicament for enhancing defensive factors include ecabet and L-glutamine each having an action of coating gastric mucosa. It is also known that *Helicobacter pylori* positive patients obtain a high therapeutic effect by eliminating the bacteria (Non-patent Document 1).

Gastrointestinal disorders may be induced by low-dose aspirin, which is a therapeutic drug in an antiplatelet therapy, and NSAID, which is prescribed to the elderly for the purpose of, for example, pain treatment of osteoarthritis. In recent years, the development of a double balloon endoscope and a capsule endoscope has increasingly discovered NSAID-induced ulcer in the small intestine. A prostaglandin formulation, a proton pump inhibitor (PPI), and the like are used as therapeutic drugs for such NSAID-induced small intestinal mucosal damages, but therapeutic effects thereof are said to be less satisfactory. Further, there is a report that NSAID-induced ulcer induces intestinal hemorrhage, and 17,000 people per year die of the disease in the United States (Non-patent Document 2).

GERD is a disease in which the esophageal mucosa is damaged by the reflux of an acid or a digestive enzyme into the esophagus due to a disorder of the reflux-preventing mechanism of the lower esophageal sphincter. However, it is known that, unlike peptic ulcers, the elimination of *Helicobacter pylori* exacerbates the condition. Further, NERD, which causes reflux symptoms such as heartburn but does not cause endoscopically-observed mucosal damage, has also started to attract attention in recent years (Non-patent Document 3).

A histamine H2 receptor inhibitor, a proton pump inhibitor, and the like are mainly used as therapeutic drugs for these acid secretion-related diseases. The histamine H2 receptor inhibitor inhibits gastric acid secretion during the night, and does not inhibit gastric acid secretion after meals. On the other hand, the proton pump inhibitor inhibits gastric acid secretion caused by histamine, gastrin, and acetylcholine, and shows higher effectiveness than the histamine H2 receptor inhibitor does. However, when these acid secretion inhibitors are administered for a long period of time, the growth of the gastric mucosa is observed, and further, the rebound of acid secretion occurs after stopping administration, resulting in the relapse of ulcers and inflammations. Further, these drugs must be carefully administered topatients with a renal or hepatic dysfunction. From the foregoing, as drugs for acid secretion-related diseases, drugs having two actions of reducing aggressive factors and enhancing defensive factors with less adverse effects even in long-term administration are desired.

Meanwhile, as appetite regulators, fenfluramine, phentermine, sibutramine, mazindol, and the like acting on the central nervous system are known, but may cause adverse effects such as dry mouth, constipation, sweating, and palpitation. Therefore, there is a demand for an appetite regulator with less adverse effects (Non-patent Document 4).

A calcium receptor is also called a calcium sensing receptor (hereinafter, also referred to as "CaSR"). The calcium receptor was cloned as a G-protein coupled seven-transmembrane receptor (G-protein coupled receptor; GPCR) capable of sensing extracellular calcium (Calcium; Ca²⁺) from bovine thyroid in 1993 (Non-patent Document 5). The calcium receptor has a function of sensing extracellular Ca²⁺ to alter the intracellular Ca²⁺ concentration, thereby regulating the production of, for example, hormones involved in the metabolic regulation of Ca²⁺, typified by parathyroid hormone. In recent years, it has been found that cinacalcet (CCT), which is a calcium receptor agonist, has an action of inhibiting parathyroid hormone secretion by acting on the calcium receptor in the parathyroid to increase the Ca²⁺ sensitivity of the calcium receptor (Non-patent Document 6). Further, it has been made clear that the calcium receptor is expressed in the kidney, brain, thyroid, bone, and gastrointestinal tract (Non-patent Document 7). It has been found that the calcium receptor is expressed in G cells and parietal cells in the stomach and has an action of promoting gastrin and gastric acid secretion (Non-patent Documents 8 and 9). Further, the calcium receptor is expressed in the large intestine to regulate water secretion (Non-patent Document 10). In view of the foregoing, it has been reported that a calcium receptor agonist can be effective for upper and lower gastrointestinal diseases (Patent Document 1).

Meanwhile, it has been demonstrated that a calcium receptor agonist regulates acid secretion and its action of inhibiting alkalization in parietal cells under histamine stimulation is comparable to that of omeprazole, which is a proton pump inhibitor (Non-patent Document 11).
As described above, the inhibiting effect of the calcium receptor agonist on an acid, which is one of the aggressive factors for the gastrointestinal mucosa, has started to become clear, but the effect on defensive factors still remains unknown.
Non-patent Document 1: Themerckmanual of medical information second home edition, online version (researched on March 13, 2008), Internet <URL http://mmh.banyu.co.jp/mmhe2j/sec09/ch121/ch121c.html>
Patent Document 1: WO 2006/123725 pamphlet
Non-patent Document 2: Nippon Rinsho 2007; Vol. 65(10): pp. 1749-1758
Non-patent Document 3: Nippon Rinsho 2007; Vol. 65(5): pp. 797-801, 841-845
Non-patent Document 4: "konnichi no chiryouyaku 2001, Explanations and Handbook", edited by Yutaka Mizushima, Nankodo Inc., p. 928
Non-patent Document 5: Brown, E. M. et al. 1993. Nature Vol. 366: pp. 575-580
Non-patent Document 6: Cohen, A. and Silverberg, S. J. 2002. Current Opinion in Pharmacology 2: pp. 734-739
Non-patent Document 7: McLarnon, S. J. and Riccardi, D. et al. 2002. European Journal of Pharmacology Vol. 447: pp. 271-278
Non-patent Document 8: Ray, J. M. et al. 1997. Journal of Clinical Investigation Vol. 99: pp. 2328-2333
Non-patent Document 9: Cheng, I. et al. 1999. Gastroenterology 1999; Vol. 116: pp. 118-126
Non-patent Document 10: Cheng, S. X. et al. 2002. The American Journal of Physiology-Gastroinstinal and Liver Physiology Vol. 283: pp. G240-G250
Non-patent Document 11: Geibel, J. P. et al. 2001. Journal of Biological Chemistry Vol. 276: pp. 39549-39552

### Disclosure of the Invention

An object of the present invention is to provide a medicament capable of preventing or treating acid secretion-related diseases, the medicament being effective for mucosal injuries induced by an acid excessively secreted in association with gastrointestinal dysfunctions or with acid secretion-related diseases, or by drugs.

The inventors of the present invention have intensively studied in order to solve the above-described problem. As a result, the inventors have found that a calcium receptor activator promotes bicarbonate secretion in the stomach and duodenum. The inventors have also confirmed that the calcium receptor activator inhibits histamine-induced gastric acid secretion. In addition, the inventors have developed a technology for isolating specific cells of the stomach, and based on this technology, have newly found that the calcium receptor is expressed in gastric D cells. In addition, the inventors have found that the calcium receptor activator promotes somatostatin secretion in gastric D cells. Based on these results, the inventors have found novel applications as prophylactic or therapeutic drugs for various acid secretion-related diseases, in which the calcium receptor activator is administered or ingested to enhance defensive factors in the stomach and duodenum. Thus, the present invention has been completed.

In addition, the inventors of the present invention have also found that the calcium receptor activator promotes somatostatin secretion in gastric D cells, while glutamic acid inhibits somatostatin secretion. The inventors have also found that, because somatostatin acts on the digestive organs, stomach, small intestine, large intestine, pancreas, and the like, and inhibits the secretion of appetite-regulating hormones and the like, the regulated use of the calcium receptor activator or the calcium receptor activator and glutamic acid can provide an appropriate appetite regulator. Thus, the present invention has been completed.

That is, the present invention relates to the following:
(1) a bicarbonate secretion promoter in the gastrointestinal tract, containing a calcium receptor activator as an active ingredient;
(2) the bicarbonate secretion promoter, wherein the calcium receptor activator is a peptide;
(3) the bicarbonate secretion promoter, wherein the peptide is selected from the group consisting of γ-Glu-X-Gly (X represents an amino acid or an amino acid derivative), γ-Glu-Val-Y (Y represents an amino acid or an amino acid derivative), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-γ-Glu-Val, γ-Glu-Val-NH₂, γ-Glu-Val-ol, γ-Glu-Ser, γ-Glu-Tau, γ-Glu-Cys (S-Me) (O), γ-Glu-Leu, γ-Glu-Ile, γ-Glu-t-Leu, and γ-Glu-Cys(S-Me);
(4) the bicarbonate secretion promoter, wherein X is Cys, Cys(SNO), Cys(S-allyl), Gly, Cys(S-Me), Abu, or Ser, and Y is Gly, Val, Glu, Lys, Phe, Ser, Pro, Arg, Asp, Met, Thr, His, Orn, Asn, Cys, or Gln;
(5) the bicarbonate secretion promoter, wherein the calcium receptor activator is selected from a compound represented by the following formula (1) or (2) and salts thereof;

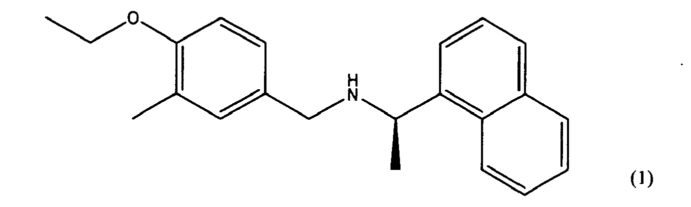

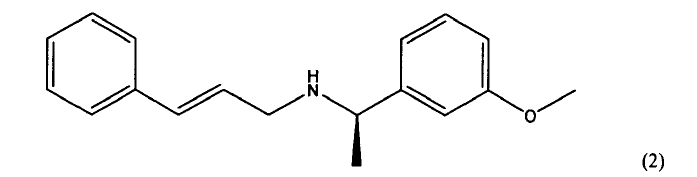

(6) the bicarbonate secretion promoter, wherein the peptide is γ-Glu-Val-Gly;
(7) the bicarbonate secretion promoter, wherein the calcium receptor activator is cinacalcet;
(8) the bicarbonate secretion promoter, which is a pharmaceutical used for the prophylaxis or treatment of an acid secretion-related disease;
(9) the bicarbonate secretion promoter, wherein the acid secretion-related disease is selected from the group consisting of gastric ulcer, duodenal ulcer, non-steroidal anti-inflammatory drug-inducedulcer, gastro-esophageal reflux disease, and non-erosive reflux disease;
(10) a pharmaceutical used for the prophylaxis or treatment of non-steroidal anti-inflammatory drug-induced ulcer, containing γ-Glu-Val-Gly or cinacalcet as an active ingredient;
(11) the bicarbonate secretion promoter, which is administered in an amount of 0.001 to 10 g/kg body weight per day for adults in terms of the amount of the calcium receptor activator;
(12) a food containing the bicarbonate secretion promoter;
(13) an appetite regulator comprising, as components, a somatostatin secretion promoter containing a calcium receptor activator as an active ingredient and an appetite stimulator, wherein one of the components is alternatively selected during use;
(14) the appetite regulator, wherein the appetite stimulator is sodium L-glutamate;

(15) a use of a calcium receptor activator for manufacturing a bicarbonate secretion promoter in the gastrointestinal tract;
(16) the use, wherein the calcium receptor activator is a peptide;
(17) the use, wherein the peptide is selected from the group consisting of γ-Glu-X-Gly (X represents an amino acid or an amino acid derivative), γ-Glu-Val-Y (Y represents an amino acid or an amino acid derivative), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me);
(18) the use, wherein X is Cys, Cys(SNO), Cys(S-allyl), Gly, Cys(S-Me), Abu, or Ser, and Y is Gly, Val, Glu, Lys, Phe, Ser, Pro, Arg, Asp, Met, Thr, His, Orn, Asn, Cys, or Gln;
(19) the use, wherein the calcium receptor activator is selected from a compound represented by the formula (1) or (2) and salts thereof;
(20) the use, wherein the peptide is γ-Glu-Val-Gly;
(21) the use, wherein the calcium receptor activator is cinacalcet;

(22) a use of the calcium receptor activator for manufacturing a pharmaceutical used for the prophylaxis or treatment of an acid secretion-related disease;
(23) the use, wherein the acid secretion-related disease is selected from the group consisting of gastric ulcer, duodenal ulcer, non-steroidal anti-inflammatory drug-induced ulcer, gastro-esophageal reflux disease, and non-erosive reflux disease;
(24) a use of γ-Glu-Val-Gly or cinacalcet for manufacturing a pharmaceutical used for the prophylaxis or treatment of non-steroidal anti-inflammatory drug-induced ulcer;
(25) a use of the calcium receptor activator and the appetite stimulator for manufacturing an appetite regulator;
(26) the use, wherein the appetite stimulator is sodium L-glutamate;

(27) a method for promoting bicarbonate secretion in the gastrointestinal tract, comprising administering a calcium receptor activator to a subject in need of the promotion of bicarbonate secretion in the gastrointestinal tract;
(28) the method for promoting bicarbonate secretion, wherein the calcium receptor activator is a peptide;
(29) the method for promoting bicarbonate secretion, wherein the peptide is selected from the group consisting of γ-Glu-X-Gly (X represents an amino acid or an amino acid derivative), γ-Glu-Val-Y (Y represents an amino acid or an amino acid derivative), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me);
(30) the method for promoting bicarbonate secretion, wherein X is Cys, Cys (SNO), Cys(S-allyl), Gly, Cys(S-Me), Abu, or Ser, and Y is Gly, Val, Glu, Lys, Phe, Ser, Pro, Arg, Asp, Met, Thr, His, Orn, Asn, Cys, or Gln;
(31) the method for promoting bicarbonate secretion, in which the calcium receptor activator is selected from a compound represented by the formula (1) or (2) and salts thereof;
(32) the method for promoting bicarbonate secretion, wherein the peptide is γ-Glu-Val-Gly;
(33) the method for promoting bicarbonate secretion, wherein the calcium receptor activator is cinacalcet;

(34) a method for preventing or treating an acid secretion-related disease, comprising administering the calcium receptor activator to a subject in need of the prophylaxis or treatment of an acid secretion-related disease;
(35) the method for preventing or treating an acid secretion-related disease, wherein the acid secretion-related disease is selected from the group consisting of gastric ulcer, duodenal ulcer, non-steroidal anti-inflammatory drug-induced ulcer, gastro-esophageal reflux disease, and non-erosive reflux disease;
(36) a method for preventing or treating non-steroidal anti-inflammatory drug-induced ulcer, comprising administering γ-Glu-Val-Gly or cinacalcet to a subject in need of the prophylaxis or treatment of non-steroidal anti-inflammatory drug-induced ulcer;
(37) the method for preventing or treating an acid secretion-related disease, wherein the promoter is administered in an amount of 0.001 to 10 g/kg body weight per day for adults in terms of the amount of a calcium receptor activator;
(38) a method for regulating appetite, comprising alternatively selecting the calcium receptor activator or the appetite stimulator during use, and administering the selected one to a subject in need of appetite regulation; and
(39) the method for regulating appetite, wherein the appetite stimulator is sodium L-glutamate.

### Brief Description of the Drawings

FIGS. 1 illustrate the influences of cinacalcet on bicarbonate secretion in the stomach and duodenum ((A) stomach, (B) duodenum).
FIG. 2 illustrates the influence of cinacalcet on histamine-induced gastric acid secretion.
FIGS. 3 illustrate the expression amounts of somatostatin and CaSR in cell fractions of rat gastric mucosa.
FIG. 4 illustrates the somatostatin secretion amounts in the case of treating fractionated gastric mucosal D cells with a CaSR agonist.
FIG. 5 illustrates histamine-induced gastric acid secretion in the case of using cinacalcet and a somatostatin receptor 2 inhibitor in combination for histamine-induced gastric acid secretion.
FIG. 6 illustrates the effect of cinacalcet on NSAID-induced enteritis.
FIG. 7 illustrates the effect of γ-EVG on NSAID-induced enteritis.
FIG. 8 illustrates the influence of γ-EVG on the bicarbonate secretion amount in the duodenum.
FIG. 9 illustrates the somatostatin secretion amounts in the case of treating fractionated gastric mucosal D cells with a CaSR agonist and sodium glutamate.

### Description of the Preferred Embodiments

Hereinafter, the present invention is described in detail.
A bicarbonate secretion promoter in the gastrointestinal tract of the present invention contains a calcium receptor activator as an active ingredient.

The term "calcium receptor" as used herein refers to a receptor that is called Calcium Sensing Receptor (CaSR) and belongs to class C of the seven-transmembrane receptors. The term "CaSR activator" as used herein includes a substance that regulates the functions of CaSR-expressing cells by binding to the above-described CaSR and activating the CaSR (hereinafter, also referred to as "CaSR agonist"), and a substance that functions to extend the CaSR activity by binding to the above-described CaSR and activating the CaSR (hereinafter , also referred to as "CaSR modulator"). Further, the term "CaSR activation" as used herein means that a ligand binds to a calcium receptor to activate a guanine nucleotide binding protein, and thereby to transduce a signal. In addition, transduction of this signal by the calcium receptor is referred to as "CaSR activity".

A calcium receptor activator can be obtained by the screening methods described below, for example, but the methods are not limited to these.

A CaSR agonist and modulator can be screened by examining the presence or absence of CaSR activation by a test substance by using CaSR-expressing cells.
The presence or absence of CaSR activation can be examined by measuring the amount of a substance (ligand) that binds to CaSR, a substance that inhibits a reaction with a signal for regulating the CaSR activity, a substance (such as a second messenger) that transduces a signal generated by the binding of a ligand to CaSR, or the like. For example, CaSR activation can be detected by detecting a second messenger generated by the binding of a ligand such as Ca²⁺ to CaSR. Further, CaSR activation can also be detected by using a labeled known ligand, and measuring the binding of the labeled ligand to CaSR.

CaSR to which a ligand has been bound acts on a GTP-binding protein (also referred to as a G protein, such as Gi or Gq) to control various cell functions via a second messenger such as cAMP. Of those, Gq activation increases the intracellular calcium concentration. Further, in the downstream of the increase in intracellular calcium concentration in the signal transduction, functions are acutely regulated through the activation of intracellular enzymes such as calmodulin, protein kinase C, and adenylate cyclase and through the phosphorylation of cytoplasmic proteins/cell membrane proteins. The activation of those intracellular enzymes causes an alteration in channel function present in a cell membrane. Thus, the presence or absence of CaSR activation by a test substance can be detected by bringing the test substance into contact with CaSR-expressing cells, and observing G protein activation using a measured value of the intracellular calcium concentration, the intracellular cAMP accumulation amount, the channel function (such as the extracellular proton production amount), the gastrointestinal hormone secretion amount, or the like as an indicator.

CaSR-expressing cells to be used for screening can be, for example, cells derived from animals including mammals such as mice, rats, hamsters, guinea pigs, rabbits, dogs, monkeys, and human beings, and avian species such as chickens. For example, gastrointestinal hormone-producing cells derived from the above-described animals are preferably used. Further, the origin of CaSR is not particularly limited, and CaSR derived from the above-described animals is exemplified. Specifically, preferred examples can include the human CaSR encoded by the human CaSR gene registered under GenBankAccession No NM_000388. It should be noted that the CaSR is not limited to the protein encoded by the gene having the above-described sequence, and may be a protein encoded by a gene having a homology of 60% or more, preferably 80% or more, more preferably 90% or more, and particularly preferably 98% or more, to the above-described sequence as long as the gene encodes a protein having the CaSR function. The GPRC6A receptor or 5.24 receptor is also known as a subtype of the CaSR, and can be used in the method below.

The test substance in screening can be any of the known compounds and novel compounds, and examples include nucleic acids, saccharides, lipids, proteins, peptides, organic low-molecular-weight compounds, compound libraries prepared by using combinatorial chemistry technology, random peptide libraries prepared by solid-phase synthesis or a phage display method, and natural ingredients derived from microorganisms, animals and plants, marine organisms or the like, and the like.

A first screening method (hereinafter, also referred to as "method A") comprises, for example, the following steps (a), (b), and (c):
(a) a step of bringing CaSR-expressing cells into contact with a test substance;
(b) a step of measuring the G protein activation in the cells brought into contact with the test substance, and comparing such activation with activation in control cells not brought into contact with the test substance; and
(c) a step of selecting a substance capable of activating CaSR based on the comparison results in the above-described (b).

In the step (a) of the above-described screening method, the CaSR-expressing cells are kept under a contact condition with the test substance. The contact of the test substance to the cells can be performed in a culture medium. The culture medium is appropriately selected depending on the kind of cells to be used and the like.

In the step (b), firstly, the activation of G proteins in the CaSR-expressing cells in the presence of the test substance is evaluated. Next, such activation is compared with activation in the absence of the test substance. Here, examples of an indicator for measuring the activation of G proteins include the intracellular calcium concentration, the amount of intracellular cAMP, the extracellular proton amount, the intracellular gastrointestinal hormone (such as somatostatin) secretion amount, and the like.

In the step (c), the comparison of the activations is conducted based on, for example, the presence or absence of a significant difference. As a result of the evaluation, if it can be confirmed that the activation is increased or extended in the presence of the test substance as compared to that in the absence of the test substance, the test substance can be assessed as a CaSR agonist.

Also, in the case of screening a CaSR modulator, in the above-described step (a), the test substance and a CaSR agonist were brought into contact with the CaSR-expressing cells; in the step (b), the activation of G proteins in cases where the CaSR agonist was brought into contact with the cells in the presence of the test substance is compared with the activation of G proteins in cases where the CaSR agonist was brought into contact with the cells in the absence of the test substance; and in the step (c), a substance which extends the activation of G proteins can be selected as a CaSR modulator.

A second screening method for a CaSR agonist or modulator comprises, for example, the following steps (a), (b), and (c):
(a) a step of bringing a test substance and a ligand acting on CaSR into contact with CaSR-expressing cells;
(b) a step of measuring the amount of the ligand bound to the cell membrane of the cells, and comparing such an amount of the ligand with the amount of ligand in control cells not brought into contact with the test substance; and
(c) a step of selecting a substance capable of activating CaSR based on the comparison results in the above-described (b).

In the step (a) of the above-described screening method, the CaSR-expressing cells are kept under a contact condition with the test substance and the ligand acting on CaSR. The contact of the test substance and the ligand acting on CaSR to the cells can be performed in a culture medium. The culture medium is appropriately selected depending on the kind of cells to be used and the like.

In the step (b), firstly, the amount of the ligand bound to the cell membrane of the CaSR-expressing cells in the presence of the test substance is evaluated. Next, such an amount of the ligand is compared with the amount of the ligand in the absence of the test substance. The amount of the ligand bound can be measured, for example, by using a radio-labeled ligand or the like.

In the step (c), the comparison of the amounts of the ligand is conducted based on, for example, the presence or absence of a significant difference. As a result of the evaluation, if it can be confirmed that the amount of the ligand bound is decreased in the presence of the test substance as compared to that in the absence of the test substance, the test substance can be assessed as a CaSR agonist or a CaSR modulator.

In addition, as for the substance which has been confirmed to decrease the amount of the ligand bound, by the above-described screening method A, it can be confirmed that the substance is a CaSR agonist.

The ligand acting on CaSR is not particularly limited, and examples of the ligand include Ca²⁺, cinacalcet, and the like.

Hereinafter, specific methods (1) to (6) for detecting a substance capable of improving gastrointestinal dysfunctions by using CaSR-expressing cells (cells having CaSR in the state where the CaSR can function) will be exemplified.

(1) A method comprising the following steps (a), (b), and (c):
(a) a step of bringing CaSR-expressing cells into contact with a test substance for a certain period of time;
(b) a step of measuring somatostatin released from the cells brought into contact with the test substance, and comparing such a released amount with the released amount in control cells not brought into contact with the test substance; and
(c) a step of selecting a substance capable of activating CaSR based on the comparison results in the above-described (b).

(2) A method comprising the following steps (a), (b), and (c):
(a) a step of bringing CaSR-expressing cells into which a calcium sensitive dye (such as Fura-2, Indo-1, or Fluo-3) has been introduced into contact with a test substance for a certain period of time;
(b) a step of measuring the fluorescence intensity (intracellular calcium concentration) in the cells brought into contact with the test substance, and comparing such an intensity with the intensity in control cells not brought into contact with the test substance; and
(c) a step of selecting a substance capable of activating CaSR based on the comparison results in the above-described (b).

Here, in the step (a) of the above-described screening method, the CaSR-expressing cells are preferably gastrointestinal hormone-producing cells which express CaSR receptor. For example, a target substance is searched on the basis of an alteration in the fluorescence intensity (intracellular calcium concentration) in the case of bringing gastrointestinal hormone-producing cells into which a calcium sensitive dye has been introduced into contact with the test substance for a certain period of time. In the case of screening a CaSR modulator, the test substance and a CaSR agonist can be brought into contact with CaSR-expressing cells into which a calcium sensitive dye (such as Fura-2, Indo-1, or Fluo-3) has been introduced.

In the step (b) of the above-described screening method, whether or not the fluorescence intensity (intracellular calcium concentration) in CaSR-expressing cells varies in the presence of the test substance is evaluated. This can be evaluated by comparing such a measured fluorescence intensity (intracellular calcium concentration) with the fluorescence intensity (intracellular calcium concentration) in the absence of the test substance. Such fluorescence intensities can be measured by using a known method. In the case of screening a CaSR modulator, the fluorescence intensity in the case of bringing a CaSR agonist into contact with the cells in the presence of the test substance can be compared with the fluorescence intensity in the case of bringing the CaSR agonist into contact with the cells in the absence of the test substance.

In step (c) of the above-described screening method, the comparison of the fluorescence intensities is conductedbased on, for example, the presence or absence of a significant difference. As a result of the evaluation of the fluorescence intensity, if it can be confirmed that the intracellular calcium concentration increases, the test substance can be assessed as a CaSR agonist. In the case of screening a CaSR modulator, a substance which extends the variation range of fluorescence intensity can be selected as a CaSR modulator.

(3) A method comprising the following steps (a), (b), and (c):
(a) a step of bringing CaSR-expressing cells into contact with a test substance for a certain period of time;
(b) a step of measuring the cAMP amount in the cells brought into contact with the test substance, and comparing such a cAMP amount with the cAMP amount in control cells not brought into contact with the test substance; and
(c) a step of selecting a substance capable of activating CaSR based on the comparison results in the above-described (b).

The cAMP amount can be measured with a commercially available assay kit.
In the step (a) of the above-described screening method, in the case of screening a CaSR modulator, the test substance and a CaSR agonist can be brought into contact with the CaSR-expressing cells.
In the step (b) of the above-described screening method, in the case of screening a CaSR modulator, the cAMP amount in the case of bringing a CaSR agonist into contact with the cells in the presence of the test substance can be compared with the cAMP amount in the case of bringing the CaSR agonist into contact with the cells in the absence of the test substance.

In the step (c) of the above-described screening method, the comparison of the cAMP amounts is conducted based on, for example, the presence or absence of a significant difference. As a result of the evaluation of the cAMP amount, if it can be confirmed that the cAMP amount increases, the test substance can be assessed as a substance capable of activating CaSR. In the case of screening a CaSR modulator, a substance which extends the increase in the cAMP amount can be selected as a CaSR modulator.

(4) A method comprising the following steps (a), (b), and (c):
(a) a step of bringing a test substance and a known ligand (such as Ca²⁺ or cinacalcet) acting on CaSR into contact with CaSR-expressing cells for a certain period of time;
(b) a step of measuring the amount of the ligand bound to the cell membrane of the cells, and comparing such an amount of the ligand with the amount of the ligand in control cells not brought into contact with the test substance; and
(c) a step of selecting a substance capable of activating CaSR based on the comparison results in the above-described (b).

The amount of the known ligand can be measured by subjecting a part of those substances to radioactive labeling, and determining the amount of radioactivity bound to the cell membrane.
In the step (c) of the above-described screening method, the comparison of the amounts of the ligand is conducted based on, for example, the presence or absence of a significant difference. As a result of the evaluation of the amount of the ligand, if it can be confirmed that the amount of the ligand bound decreases, the test substance can be assessed as a CaSR agonist or modulator.

(5) A method comprising the following steps (a), (b), and (c):
(a) a step of bringing CaSR-expressing cells into which a cAMP sensitive fluorescent protein (such as FICRhR) has been introduced into contact with a test substance for a certain period of time;
(b) a step of measuring the fluorescence intensity (intracellular cAMP concentration) in the cells brought into contact with the test substance, and comparing such an intensity with the intensity in control cells not brought into contact with the test substance; and
(c) a step of selecting a substance capable of activating CaSR based on the comparison results in the above-described (b).

Here, the CaSR-expressing cells are preferably gastrointestinal hormone-producing cells which express CaSR.

In the step (a) of the above-described screening method, in the case of screening a CaSR modulator, the test substance and a CaSR agonist can be brought into contact with the CaSR-expressing cells into which a cAMP sensitive fluorescent protein (such as FICRhR) has been introduced.

In the step (b) of the above-described screening method, in the case of screening a CaSR modulator, the fluorescence intensity (intracellular cAMP concentration) in the case of bringing a CaSR agonist into contact with the cells in the presence of the test substance can be compared with the fluorescence intensity (intracellular cAMP concentration) in the case of bringing the CaSR agonist into contact with the cells in the absence of the test substance.

In the step (c) of the above-described screening method, the comparison of the fluorescence intensities is conducted based on, for example, the presence or absence of a significant difference. As a result of the evaluation of the fluorescence intensity, if it can be confirmed that the fluorescence intensity increases, the test substance can be assessed as a CaSR agonist. In the case of screening a CaSR modulator, a substance which extends the increase in the fluorescence intensity can be selected as a CaSR modulator.

(6) A method comprising the following steps (a), (b), and (c):
(a) a step of bringing CaSR-expressing cells into contact with a test substance for a certain period of time;
(b) a step of measuring the extracellular proton production amount in the cells brought into contact with the test substance, and comparing such a proton production amount with the extracellular proton production amount in control cells not brought into contact with the test substance; and
(c) a step of selecting a substance capable of activating CaSR based on the comparison results in the above-described (b).

Here, the CaSR-expressing cells are preferably gastrointestinal hormone-producing cells which express CaSR. For example, a target substance can be detected by measuring the extracellular proton production amount in the case of bringing gastrointestinal hormone cells which express CaSR into contact with a CaSR agonist and a test substance for a certain period of time, and using the proton production amount as an indicator. The proton production amount is measured with a cytosensor.

In the step (a) of the above-described screening method, in the case of screening a CaSR modulator, the test substance and a CaSR agonist can be brought into contact with the CaSR-expressing cells.

In the step (b) of the above-described screening method, in the case of screening a CaSR modulator, the extracellular proton production amount in the case of bringing a CaSR agonist into contact with the cells in the presence of the test substance can be compared with the extracellular proton production amount in the case of bringing the CaSR agonist into contact with the cells in the absence of the test substance.

In the step (c) of the above-described screening method, the comparison of the proton production amounts is conducted based on, for example, the presence or absence of a significant difference. As a result of the evaluation of the proton production amount, if it can be confirmed that the extracellular proton production amount increases, the test substance can be assessed as a CaSR agonist. In the case of screening a CaSR modulator, a substance which extends the increase in the extracellular proton production amount can be selected as the CaSR modulator.

(7) A method comprising the following steps (a), (b), and (c):
   (a) a step of bringing CaSR-expressing cells into contact with a test substance for a certain period of time;
   (b) a step of measuring the amount of gastrointestinal hormone secretion in the cells which is in contact with the test substance, and comparing the amount of gastrointestinal hormone secretion with those of the gastrointestinal hormone secretion in control cells without the test substance; and
   (c) a step of selecting a substance capable of activating CaSR based on the comparison results in the above-described (b).

Here, the CaSR-expressing cells are preferably gastrointestinal hormone-producing cells expressing CaSR. For example, a target substance can be searched by measuring the amount of gastrointestinal hormone secretion in the case of bringing gastrointestinal hormone-producing cells expressing CaSR into contact with a CaSR agonist and a test substance for a certain period of time, and using the amount of gastrointestinal hormone secretion as an indicator. The amount of gastrointestinal hormone secretion can be measured by using a commercially available assay kit.

In the step (a) of the above-described screening method, in the case of screening a CaSR modulator, the test substance and a CaSR agonist can be brought into contact with the CaSR-expressing cells.

In the step (b) of the above-described screening method, in the case of screening a CaSR modulator, the amount of gastrointestinal hormone secretion in the case of bringing a CaSR agonist into contact with the cells in the presence of the test substance can be compared with the amount of gastrointestinal hormone secretion in the case of bringing the CaSR agonist into contact with the cells in the absence of the test substance.

In the step (c) of the above-described screening method, the comparison of the gastrointestinal hormone secretion amounts is conducted based on, for example, the presence or absence of a significant difference. As a result of the evaluation of the gastrointestinal hormone secretion amount, if it can be confirmed that the gastrointestinal hormone secretion amount varies, the test substance can be assessed as a CaSR agonist. In the case of screening a CaSR modulator, a substance which extends the variation range of the gastrointestinal hormone secretion amount can be selected as a CaSR modulator.

The CaSR activator (CaSR agonist or modulator) obtained as above is preferably confirmed to have an action of bicarbonate secretion promoter in the gastrointestinal tract. The action of promoting bicarbonate secretion can be confirmed in accordance with, for example, the methods of Aihara et al. (Aihara, E. et al. 2005. J. Pharmacol. Exp. Ther. 315: 423-432) and Kagawa et al. (Kagawa, S. et al. 2003. Digestive Diseases and Sciences 48: 1850-1856) described in the Examples.

Specific examples of the CaSR agonist include: cations each having two or more valences such as calcium and gadolinium; amino acids such as phenylalanine and tryptophan; various peptides such as γ-Glu-X-Gly (X represents an amino acid or an amino acid derivative), γ-Glu-Val-Y (Y represents an amino acid or an amino acid derivative), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me); basic peptides such as polylysine; polyamines such as spermine and spermidine; proteins such as protamine; and various low-molecular-weight compounds such as cinacalcet and the compound represented by the chemical formula (1) ((R)-N-[(4-ethoxy-3-methylphenyl)methyl]-1-(1-naphthyl)ethy lamine) or the compound represented by the chemical formula (2) ((R)-N-(3-phenylprop-2-enyl)-1-(3-methoxyphenyl)ethylamine).

It should be noted that, in this description, each of amino acids which constitute a peptide is in the L-form unless otherwise stated. Herein, examples of the amino acid include: a neutral amino acid such as Gly, Ala, Val, Leu, Ile, Ser, Thr, Cys, Met, Asn, Gln, Pro, or Hyp; an acidic amino acid such as Asp or Glu; a basic amino acid such as Lys, Arg, or His; an aromatic amino acid such as Phe, Tyr, or Trp; and homoserine, citrulline, ornithine, α-aminobutyric acid, norvaline, norleucine, and taurine.

In this description, abbreviations for amino group residues mean the following amino acids.
- (1): Gly: Glycine
- (2): Ala: Alanine
- (3): Val: Valine
- (4): Leu: Leucine
- (5): Ile: Isoleucine
- (6): Met: Methionine
- (7): Phe: Phenylalanine
- (8): Tyr: Tyrosine
- (9): Trp: Tryptophan
- (10): His: Histidine
- (11): Lys: Lysine
- (12): Arg: Arginine
- (13): Ser: Serine
- (14): Thr: Threonine
- (15): Asp: Aspartic acid
- (16): Glu: Glutamic acid
- (17): Asn: Asparagine
- (18): Gln: Glutamine
- (19): Cys: Cysteine
- (20): Pro: Proline
- (21): Orn: Ornithine
- (22): Sar: Sarcosine
- (23): Cit: Citrulline
- (24): N-Val: Norvaline
- (25): N-Leu: Norleucine
- (26): Abu: α-Aminobutyric acid
- (27): Tau: Taurine
- (28): Hyp: Hydroxyproline
- (29): t-Leu: tert-Leucine

Further, the amino acid derivative represents any one of various derivatives of the above-described amino acids, and examples thereof include an unusual amino acid, a non-natural amino acid, an amino alcohol, and an amino acid in which an amino acid side chain such as the terminal carbonyl group, the terminal amino group, or the thiol group of cysteine is replaced with any one of various substituents. Examples of the substituents include an alkyl group, an acyl group, a hydroxy group, an amino group, an alkylamino group, a nitro group, a sulfonyl group, and various protection groups. Examples of the substituted amino acid include: Arg(NO₂): N-γ-nitroarginine; Cys(SNO): S-nitrocysteine; Cys(S-Me): S-methylcysteine; Cys(S-allyl): S-allylcysteine; Val-NH₂: valinamide; and Val-ol: valinol (2-amino-3-methyl-1-butanol).

It should be noted that the " (O) in γ-Glu-Met (O) and γ-Glu-Cys(S-Me)(O) indicates a sulfoxide structure. The "γ: gamma" in γ-Glu indicates that the glutamic acid binds to another amino acid via the carboxy group at the γ position of the glutamic acid.

In the peptide, "X" is preferably Cys, Cys(SNO), Cys (S-allyl), Gly, Cys (S-Me), Abu, or Ser, and "Y" is preferably Gly, Val, Glu, Lys, Phe, Ser, Pro, Arg, Asp, Met, Thr, His, Orn, Asn, Cys, or Gln. However, "X" and "Y" are not limited thereto. A preferred example of the peptide is γ-Glu-Val-Gly.

A commercially available product can be used as the above-described peptide. Further, the peptide can be obtained by appropriately employing a known technique such as (1) a chemical synthesis method or (2) a synthesis method through an enzymatic reaction. The peptide to be used in the present invention contains 2 to 3 amino acid residues, i.e., is relatively short, and hence, the chemical synthesis method is convenient. In the case of the chemical synthesis, the oligopeptide can be synthesized or semi-synthesized by using a peptide synthesizer. An example of the chemical synthesis method is a peptide solid phase synthesis method. The peptide synthesized as described above can be purified by general means such as ion exchange chromatography, reversed phase high performance liquid chromatography, or affinity chromatography. Such peptide solid phase synthesis method and the subsequent peptide purification are well known in the technical field.

Further, the peptide to be used in the present invention can also be produced by an enzymatic reaction. For example, the method described in WO 2004/011653 A1 can be employed. That is, the peptide can also be produced by allowing one amino acid or dipeptide in which the carboxyl terminus of the amino acid or dipeptide is esterified or amidated and an amino acid having a free amino group (for example, an amino acid whose carboxyl group is protected) to react with each other in the presence of a peptide-producing enzyme, and purifying the produced dipeptide or tripeptide.
Examples of the peptide-producing enzyme include a culture of a microorganism having an ability to produce a peptide, microbial cells separated from the culture, a processed product of the microbial cells, and a peptide-producing enzyme derived from the microorganism.

It should be noted that the peptide to be used in the present invention is not only produced by such enzymatic method or chemical synthesis method as described above, but also may exist in, for example, a plant such as a vegetable or a fruit, a microorganism such as a yeast, and a yeast extract. When the peptide exists in natural products, the peptide extracted from these natural products can be used.

Further, the peptide does not need to be isolated before use, and a fraction containing the peptide of the present invention in a large amount can also be used. A example includes a yeast extract containing glutathione (γ-Glu-Cys-Gly) or a fraction thereof. The preparation of the yeast extract or the like can be conducted in the same manner as general yeast extract preparation. The yeast extract can be a treated product of yeast cells extracted with hot water, or can be a treated product of digested yeast cells. The fraction of the yeast extract is not particularly limited as long as the fraction contains glutathione.

The compound represented by the chemical formula (1) and the compound represented by the chemical formula (2) can be synthesized by, for example, such a known method as described in US 6211244 A. Further, a commercially available product thereof can also be used.

There is described in WO2007/055388, WO2007/055388, WO 2008/139945, WO 2008/139946, WO 2008/139947 that each of the above-described amino acids and peptides has an action of activating CaSR.

In addition, examples of the CaSR activator include compounds described in US Patent 6211244, WO 06/123725, WO 05/115975, US Patent 6313146, US Patent 6213146, US Patent 5688938, US Patent 5763569, US Patent 5858684, US Patent 5962314, US Patent 6001884, US Patent 6011068, US Patent 6031003, WO 95/11221, WO 1996/012697, WO 2002/059102, and JP 1999-130737 A.
Further, a particularly preferred example of the CaSR activator is cinacalcet. Cinacalcet can be synthesized by a known method, and a commercially available product thereof can also be used.
Further, preferred examples of the CaSR activator include the following compounds:
(R)-N-[3-(4-methylphenyl)prop-2-enyl]-1-(3-methoxyphe nyl)ethylamine,
(R)-N-[3-(2-methylphenyl)prop-2-enyl]-1-(3-methoxyphe nyl)ethylamine,
(R)-N-[3-(2,4,6-trimethylphenyl)prop-2-enyl]-1-(3-met hoxyphenyl)ethylamine,
(R)-N-[3-(4-isopropylphenyl)prop-2-enyl]-1-(3-methoxy phenyl)ethylamine,
(R)-N-[3-(2,4-dimethylphenyl)prop-2-enyl]-1-(3-methox yphenyl)ethylamine,
(R)-N-[3-(3-methylphenyl)prop-2-enyl]-1-(3-methoxyphe nyl)ethylamine,
(R)-N-(2-methyl-3-phenylprop-2-enyl)-1-(3-methoxyphen yl)ethylamine,
(R,R)-N-(2-methyl-4-phenylbut-3-enyl)-1-(3-methoxyphe nyl)ethylamine,
(S,R)-N-(2-methyl-4-phenylbut-3-enyl)-1-(3-methoxyphe nyl)ethylamine,
(R)-N-[3-(3-trifluoromethoxyphenyl)propyl]-1-(3-metho xyphenyl)ethylamine,
(R)-N-[(R)-3-(3-trifluoromethylphenyl)-1-methylpropyl ]-1-(3-methoxyphenyl)ethylamine,
(R)-N-[(R)-3-(3-trifluoromethoxyphenyl)-1-methylpropy 1]-1-(1-naphthyl)ethylamine).

The bicarbonate secretion promoter of the present invention contains the CaSR activator as an active ingredient. One kind of CaSR activator can be contained alone, or two or more kinds thereof can be contained.

In the case of using plural kinds of the CaSR activators as a mixture, if one kind thereof is a peptide or a low-molecular-weight compound, at least one kind of the others is preferably a cation such as calcium and gadolinium or more preferably calcium.

It should be noted that a part of or all of the CaSR activator can also be used in the form of a salt as well as a free compound. The CaSR activator as used herein is a concept that encompasses both a free compound and a salt thereof. Examples of the form of the salt include acid addition salts and salts with a base, and it is preferred to select a salt acceptable as a pharmaceutical or a food. A compound capable of forming the salt acceptable as a pharmaceutical or a food or beverage is, for example, an inorganic salt such as a hydrochloride, a hydrobromide, a sulfate, or a phosphate, or an organic salt such as an acetate, a lactate, a citrate, a tartrate, a maleate, a fumarate, or a monomethyl sulfate.

The bicarbonate secretion promoter of the present invention promotes bicarbonate secretion in the gastrointestinal tract. Examples of the gastrointestinal tract include the stomach and small intestine (duodenum, jejunum, and ileum). In particular, the bicarbonate secretion promoter remarkably promotes bicarbonate secretion in the stomach and duodenum. The bicarbonate secretion promoter of the present invention can be used as a pharmaceutical for the treatment or prophylaxis of acid secretion-related diseases, for example. The treatment as used herein is a concept that encompasses the improvement of conditions or symptoms and the prophylaxis of progression (exacerbation) of conditions or symptoms.

Examples of the "acid secretion-related diseases" as used herein include diseases which are induced by the exposure to gastric acid, a digestive enzyme, or a drug such as NSAID in the esophagus, stomach, and small intestine (duodenum, jejunum, and ileum), and which can be treated or prevented by promoting bicarbonate secretion. Examples of the acid secretion-related diseases in the present invention include gastric ulcer, duodenal ulcer, NSAID-induced ulcer, GERD, and NERD. The bicarbonate secretion promoter of the present invention is particularly preferable for the treatment or prophylaxis of NSAID-induced ulcer. Of those, γ-Glu-Val-Gly and cinacalcet are preferable for the treatment or prophylaxis of NSAID-induced ulcer. The present invention includes, for example, the following:
a therapeutic or prophylactic agent for NSAID-induced ulcer containing γ-Glu-Val-Gly as an active ingredient; and
a therapeutic or prophylactic agent for NSAID-induced ulcer containing cinacalcet as an active ingredient.

The bicarbonate secretion promoter of the present invention can serve as a more effective therapeutic drug by being used in combination with various medicaments each having an action of inhibiting aggressive factors, such as a histamine H2 receptor antagonist (H2 blocker) and a proton pump inhibitor. Examples of the histamine H2 receptor antagonist include, but are not limited to, cimetidine, ranitidine, famotidine, and nizatidine, and examples of the proton pump inhibitor include, but are not limited to, omeprazole, lansoprazole, and rabeprazole.

Further, as described above, the inventors of the present invention have first found that the CaSR activator promotes somatostatin secretion. Thus, another embodiment of the present invention is a somatostatin secretion promoter containing the CaSR activator as an active ingredient. Somatostatin is known to inhibit gastric acid secretion, for example. Accordingly, the somatostatin secretion promoter of the present invention can be usefully used for the attenuation of aggressive factors.

The somatostatin secretion promoter of the present invention can also be used as an appetite inhibitor. Thus, the somatostatin secretion promoter of the present invention and an appetite stimulator are used in combination to provide an appetite regulator. The "appetite regulation" as used herein refers to enhancing the appetite of people with anorexia, and leading people with an overeating tendency to normal eating behavior, and the appetite regulator of the present invention is used for the treatment or prophylaxis thereof.

The above-described appetite regulator contains, as components, the somatostatin secretionpromoter of the present invention and the appetite stimulator, and one of these components is alternatively selected during use. That is, when appetite inhibition is intended, the somatostatin secretion promoter is selected, and when appetite stimulation is intended, the appetite stimulator is selected, before administration or ingestion. An example of the appetite stimulator is sodium glutamate.

The bicarbonate secretion promoter or appetite regulator of the present invention is useful for mammals (such as mice, rats, hamsters, rabbits, cats, dogs, cattle, sheep, monkeys, and human beings).

As for the bicarbonate secretion promoter of the present invention, there are no particular limitations on the dosage form and administration form except that the CaSR activator is contained. The administration can be oral administration or parenteral administration (ingestion) such as drip infusion administration or injection administration (transvenous administration). The oral administration is preferred because of ease of administration, but the administration is not limited thereto.

General dosage forms for pharmaceutical formulations such as a granule, a fine granule, a dust formulation, a coated tablet, a tablet, a suppository, a powder, a (micro)capsule, a chewable, a syrup, a juice, a solution, a suspension, and an emulsion as an orally-administered agent and such as a formulation for direct intravenous infusion, a formulation for drip infusion, and a formulation for prolonging the release of an active ingredient as an injection can be adopted.

In the case of oral administration, although an appropriate dosage varies depending on the symptoms and age of a patient, and a dosage method, a dosage can be any amount as long as the dosage is effective for the treatment or prophylaxis. The dosage is appropriately adjusted depending on the age, gender, body weight, symptom, and the like of a patient. For example, in the case of oral administration, the dosage is usually preferably 0.001 g to 10 g/kg body weight per day for an adult and more preferably 0.1 g to 1 g/kg body weight per day for an adult in terms of the amount of the CaSR activator.
Further, the dosage in the case of parenteral administration such as drip infusion administration or injection administration (transvenous administration) is preferably about 10- to 20-fold less than the dosage (ingestion amount) in the preferred range for the oral administration.

The above-described dosage in the oral administration can be similarly applied to a food described below as well. However, the CaSR activator can be contained in the food so that the ingestion amount can be smaller than the above-described dosage in the oral administration.

The bicarbonate secretion promoter or appetite regulator of the present invention can be formulated by a conventional method. If required for formulation, various pharmacologically acceptable substances for formulation can be blended (as assistants and the like). The substances for formulation can be appropriately selected depending on the dosage form of the formulation, and examples of the substances for formulation include an excipient, a diluent, an additive, a disintegrant, a binder, a coating agent, a lubricating agent, a sliding agent, a lubricant, a flavoring agent, a sweetener, and a solubilizer. In addition, specific examples of the substances for formulation include magnesium carbonate, titanium dioxide, lactose, mannitol, and other sugars, talc, cow's milk protein, gelatin, starch, cellulose and its derivatives, animal and vegetable oils, polyethylene glycol, and solvents such as sterile water and monohydric or polyhydric alcohols such as glycerol. It should be noted that, in the appetite regulator of the present invention, the somatostatin secretion promoter and the appetite stimulator are each individually formulated and packed into separate packages.

The bicarbonate secretion promoter or appetite regulator of the present invention can be formulated not only by a conventional method but also by various pharmaceutical formulation forms to be developed in the future. To the formulation, a method to be developed in the future can be appropriately adopted.

As for the bicarbonate secretion promoter or appetite regulator of the present invention, a document with instructions for use can be inserted in a package comprising the promoter or the regulator. An example of the document is so-called a package insert with instructions for usage, efficacy, administration method, and the like,.

The bicarbonate secretion promoter of the present invention can be incorporated into a food. The form of the food is not particularly limited, and the food can be manufactured by the same method using the same materials as for manufacturing a general food except that the CaSR activator is blended. Examples of the food include: seasonings; beverages such as juice or cows milk; confectioneries; jellies; health foods; processed agricultural products; processed fishery products; processed animal products such as cows milk; and food supplements. Further, it is also possible to provide such foods as foods with health claims. The foods with health claims also include foods bearing labels indicating that the foods are used for the prophylaxis or treatment or improvement of acid secretion-related diseases, in particular, foods for specified health uses and the like.

When the bicarbonate secretion promoter of the present invention is used as a food supplement, the promoter can be prepared in the form of a tablet, a capsule, a powder, a granule, a suspension, a chewable, a syrup, or the like. The food supplement as used herein refers to a product which is ingested for the purpose of supplementing nutrients as well as ingested as a food, and also includes a nutritious supplement, a supplement, and the like. Further, the food supplement of the present invention can include a part of foods with health claims.

### Examples

Hereinafter, the present invention is more specifically described by way of examples. However, the present invention is not limited thereto.

### [Production Example 1] Synthesis of γ-Glu-Val-Gly

Boc-Val-OH (8.69 g, 40.0 mmol) and Gly-OBzl·HCl (8.07 g, 40.0 mmol) were dissolved in methylene chloride (100 ml) and the solution was kept at 0°C. Triethylamine (6.13 ml, 44.0 mmol), HOBt (1-hydroxybenzotriazole, 6.74 g, 44.0 mmol), and WSC·HCl (1-ethyl-3-(3-dimethylaminopropyl)carbodiimide hydrochloride, 8.44 g, 44.0 mmol) were added to the solution, and the mixture was stirred overnight at room temperature. The reaction solution was concentrated under reduced pressure, and the residue was dissolved in ethyl acetate (200ml). The solution was washed with water (50 ml), a 5% citric acid aqueous solution (50ml × twice), saturated saline (50 ml), a 5% sodium bicarbonate aqueous solution (50 ml × twice), and saturated saline (50 ml). The organic layer was dried over anhydrous magnesium sulfate, magnesium sulfate was removed by filtration, and the filtrate was concentrated under reduced pressure. The residue was recrystallized from ethyl acetate-n-hexane to yield Boc-Val-Gly-OBzl (13.2 g, 36.2 mmol) as a white crystal.

Boc-Val-Gly-OBzl (5.47 g, 15.0 mmol) was added to a 4 N HCl/dioxane solution (40 ml), and the mixture was stirred at room temperature for 50 minutes. Dioxane was removed by concentration under reduced pressure, n-hexane (30 ml) was added to the residue, and the mixture was concentrated under reduced pressure. The procedure was repeated three times to quantitatively provide H-Val-Gly-OBzl·HCl.

H-Val-Gly-OBzl·HCl described above and Z-Glu-OBzl (5.57 g, 15.0 mmol) were dissolved in methylene chloride (50 ml), and the solution was kept at 0°C. Triethylamine (2. 30 ml, 16.5 mmol), HOBt (1-hydroxybenzotriazole, 2.53 g, 16.5 mmol), and WSC-HCl (1-ethyl-3-(3-dimethylaminopropyl)carbodiimide hydrochloride, 3.16 g, 16.5 mmol) were added to the solution, and the mixture was stirred at room temperature overnight for 2 days. The reaction solution was concentrated under reduced pressure, and the residue was dissolved in heated ethyl acetate (1500 ml). The solution was washed with water (200 ml), a 5% citric acid aqueous solution (200 ml × twice), saturated saline (150 ml), a 5% sodium bicarbonate aqueous solution (200 ml × twice), and saturated saline (150 ml). The organic layer was dried over anhydrous magnesium sulfate, magnesium sulfate was removed by filtration, and the filtrate was concentrated under reduced pressure. The precipitated crystal was collected by filtration and dried under reduced pressure to yield Z-Glu(Val-Gly-OBzl)-OBzl (6. 51 g, 10.5 mmol) as a white crystal.

Z-Glu(Val-Gly-OBzl)-OBzl described above (6.20 g, 10.03 mmol) was suspended in ethanol (200 ml), 10% palladium/carbon (1.50 g) was added to the suspension, and a reduction reaction was performed under a hydrogen atmosphere at 55°C for 5 hours. During the reaction, 100 ml in a total volume of water was gradually added. The catalyst was removed by filtration using a Kiriyama funnel, and the filtrate was concentrated under reduced pressure to a half volume. The reaction solution was further filtered through a membrane filter, and the filtrate was concentrated under reduced pressure. The residue was dissolved in a small volume of water, and then ethanol was added to precipitate a crystal. The crystal was collected by filtration and dried under reduced pressure to yield γ-Glu-Val-Gly as a white powder (2.85g, 9.40mmol). Hereinafter, γ-Glu-Val-Gly is also referred to as "γ-EVG."

ESI-MS: (M+H)⁺=304.1.

¹H-NMR (400 MHz, D₂O) δ (ppm): 0.87 (3H, d, J=6.8 Hz), 0.88 (3H, d, J=6.8 Hz), 1.99-2.09 (3H, m), 2.38-2.51 (2H, m), 3.72 (1H, t, J=6.35 Hz), 3.86 (1H, d, J=17.8 Hz), 3.80 (1H, d, J=17.8 Hz), 4.07 (1H, d, J=6.8 Hz).

[Production Example 2] Synthesis of (R)-N-(3-(3-trifluoromethylphenyl)propyl)-1-(1-naphthyl)eth ylamine hydrochloride (cinacalcet hydrochloride)

### (Step 1) Synthesis of

### 3-(3-trifluoromethylphenyl)-propionic acid methyl ester

A mixture of 2.20 g of 3- (trifluoromethyl) cinnamic acid, 166 mg of palladium/carbon (10%, wet), and 40 ml of ethanol was stirred overnight under a hydrogen atmosphere at 1 atm. Palladium/carbon was separated by filtration, and the filtrate was concentrated under reduced pressure. 20 ml of methanol and 4 drops of concentrated sulfuric acid were added and the mixture was stirred at 60°C for 2 hours, and then left to cool down. After concentration under reduced pressure, 20 ml of a saturated sodium bicarbonate aqueous solution was added, and the resultant was extracted with 20 ml of dichloromethane. The extract was dried over anhydrous sodium sulfate and then concentrated under reduced pressure to afford 2.40 g of the captioned compound as an oil.

¹H-NMR (300 MHz, CDCl₃) δ 2.66 (2H, t, J=7.5 Hz), 3.02 (2H, t, J=7.5 Hz), 3.68 (3H, s), 7.37-7.50 (4H, m)

### (Step 2) Synthesis of

### 3-(3-trifluoromethylphenyl)propanal

2.40 g of 3-(3-trifluoromethylphenyl)-propionic acid methyl ester synthesized in Step 1 was dissolved in 20 ml of dry dichloromethane. 13 ml of diisopropyl aluminum hydride solution (0.91 M) in hexane was dropped over 5 minutes at -78°C under an argon atmosphere, and the mixture was stirred at the same temperature for 40 minutes. 50 ml of a saturated ammonium chloride aqueous solution was dropped, and then the mixture was stirred and the temperature was raised to room temperature. 20 ml of water and 5 ml of concentrated hydrochloric acid were added, and the resultant was separated into an aqueous layer and an organic layer. The aqueous layer was extracted with dichloromethane, and the extract was combined with the separated organic layer, dried over anhydrous sodium sulfate, and then concentrated under reduced pressure to afford 2.12 g of the captioned compound as an oil.

¹H-NMR (300 MHz, CDCl₃) δ 2.83 (2H, t-t, J=7.5 Hz, 1.2 Hz), 3.02 (2H, t, J=7.5 Hz), 7.36-7.52 (4H, m), 9.83 (1H, t, J=1.2 Hz)

### (Step 3) Synthesis of

### (R)-N-(3-(3-trifluoromethylphenyl)propyl)-1-(1-naphthyl)eth ylamine hydrochloride (cinacalcet hydrochloride)

To a mixture of 2.12 g of 3-(3-trifluoromethylphenyl)propanalsynthesizedinStep2, 2.0 ml of (R)-1-(1-naphthyl)ethylamine, 3.42 g of sodium triacetoxyborohydride, and 150 ml of dry dichloromethane was added 0.750 ml of glacial acetic acid, and the mixture was stirred at room temperature for 5 hours. After 100 ml of water had been added and the mixture had been stirred for 3 hours, 100 ml of a 2M sodium hydroxide aqueous solution was added, and the resultant was separated into an aqueous layer and an organic layer. The aqueous layer was extracted with dichloromethane, and the extract was combined with the separated organic layer, dried over anhydrous sodium sulfate, and then concentrated under reduced pressure. The residue was purified with column chromatography (silica gel/hexane:ethyl acetate of 4:1 to 1:1) and then concentrated to afford 3.41 g of (R)-N-(3-(3-trifluoromethylphenyl)propyl)-1-(1-naphthyl)eth ylamine as an oil. The compound was dissolved in 10 ml of dichloromethane, 5 ml of 4M hydrochloric acid/dioxane and 20 ml of toluene were added, and the mixture was concentrated under reduced pressure to dryness. The residue was recrystallized from 40 ml of ethanol and 200 ml of heptane to afford 1.71 g of the captioned compound.

¹H-NMR (300 MHz, DMSO-d⁶) δ 1. 69 (3H, d, J=6.6 Hz), 2.00 (2H, quintet, J=7.8 Hz), 2.72 (2H, t, J=7.5 Hz), 2.65-2.85 (1H, br), 2.90-3.05 (1H, br) , 5.24-5.38 (1H, br), 7.44-7.67 (7H, m), 7.96-8.04 (3H, m), 8.23-8.28 (1H, pseud d), 9.20-9.40 (1H, br), 9.80-10.00 (1H, br) MS (ESI, m/z) 358(MH+)

### [Example 1] Effect of cinacalcet on bicarbonate secretion in stomach and duodenum

The bicarbonate secretion was evaluated in accordance with the methods of Aihara et al. (Aihara, E. et al . 2005. J. Pharmacol. Exp. Ther. 315: 423-432) and Kagawa et al. (Kagawa, S. et al. 2003. Digestive Diseases and Sciences 48: 1850-1856).

The bicarbonate secretion in the stomach was measured as described below. The abdomen of each of male SD rats was opened in the midline under anesthesia with urethane (1.25 g/kg/5 ml, intraperitoneal administration) to expose the stomach. The stomach was left to stand still in an ex-vivo chamber (Tamura Seisakusho; 3.1 cm²) and perfused with physiological saline saturated with 100% O₂ gas. In order to inhibit acid secretion, 60 mg/kg omeprazole was intraperitoneally administered. The bicarbonate secretion was continuously measured by dropping 2 mM HCl to a perfusate until an end point of pH7.0 by employing a pH-stat method (TOA, AUT-501).

As for the bicarbonate secretion in the duodenum, the abdomen of each of male SD rats was opened under anesthesia, and a duodenum loop having a length of 1.7 cm from the pyloric ring of the stomach was prepared. The lumen of the loop was perfused with physiological saline saturated with 100% O₂ gas (flow rate: 1 ml/min).

A drug was administered into the gastric or duodenal lumen after the bicarbonate secretion at the baseline had been measured for 30 minutes, and the bicarbonate secretion amount was measured every 10 minutes.

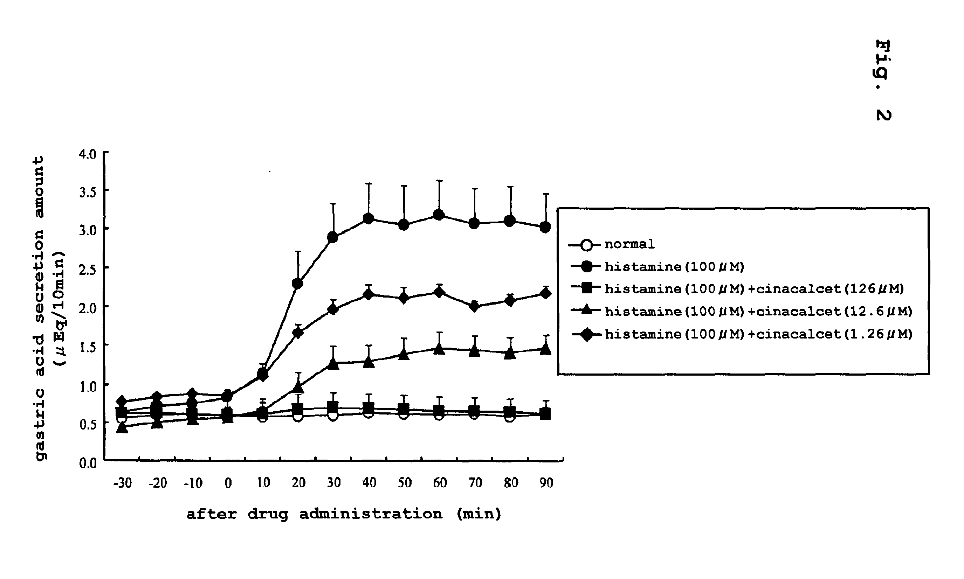

FIG. 2 illustrates the results. Cinacalcet (10 mg/kg) promoted bicarbonate secretion in the stomach and duodenum.

### [Example 2] Influence of cinacalcet on histamine-induced gastric acid secretion

A model prepared in accordance with the method reported by Horie et al. (British Journal of Pharmacology 1994; 112: 87-92) was used as a model for histamine-induced gastric acid secretion. That is, 6-week-old male ddY mice were fasted for 18 hours, and the stomach was removed from each of the mice under anesthesia with urethane (1.25g/kg/10ml, intraperitoneal administration). A polyethylene cannula was inserted at each of the pyloric and esophageal sides of the stomach, and the stomach was left to stand still in an organ bath filled with a buffer (118.1 mM NaCl, 4.8 mM KCl, 1.0 mM KH₂PO₄, 16.0 mM Na₂HPO₄, 1.2 mM MgSO₄ 0.65 mM CaCl₂, and 31. 6 mM glucose, saturated with 95% O₂/5% CO₂ gas, 37°C). The intragastric pressure was kept at 15 cmH₂O and the lumen of the stomach was perfused with a buffer (135.8 mM NaCl, 4.8 mM KCl, 1.2 mM MgSO₄ 1.3 mM CaCl₂, and 31.6 mM glucose, pH 5.4, saturated with 95% O₂/5% CO₂ gas, 37°C) at a flow rate of 1 ml/min.

The acid secretion amount was continuously measured by dropping 10 mM NaOH to a perfusate until an end point of pH 5.4 by employing a pH-stat method (TOA, AUT-501). The intragastric acid secretion amount during no stimulation was measured for 30 minutes, and then the extirpated stomach left to stand still in the organ bath was treated with 100 µM histamine and cinacalcet (1.26, 12.6, and 126 µM). The gastric acid secretion amount was titrated for up to 90 minutes. The Dunnett' s test was used as a statistical test for an effect of cinacalcet.

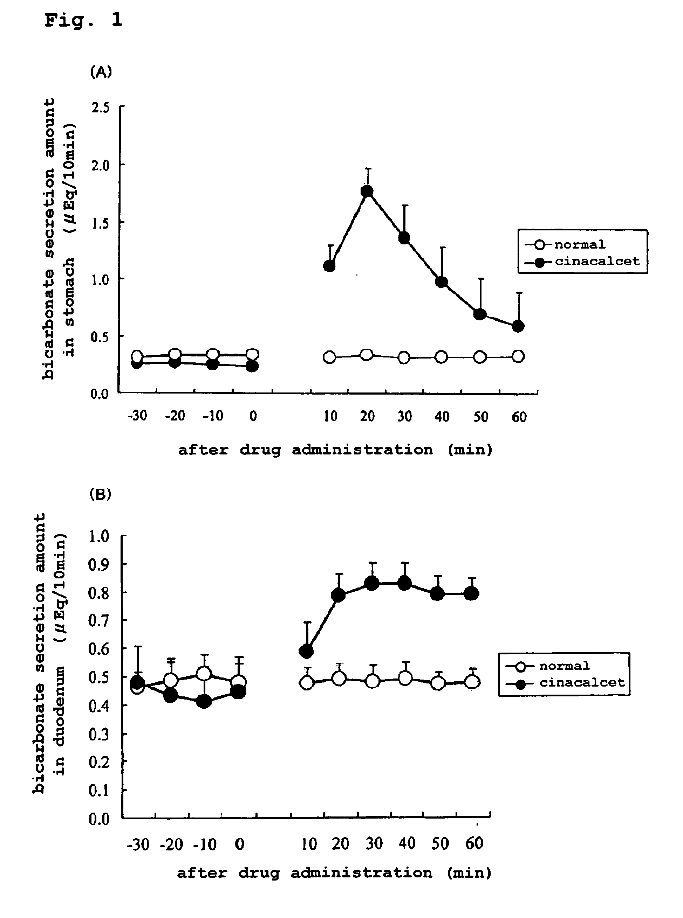

FIGS. 1 illustrate the results. The acid secretion amount, which was 0.5 to 0.8 µEq/10 min during no stimulation, increased to about 3.2 µEq/10 min by histamine stimulation. Cinacalcet inhibited histamine-induced gastric acid secretion dose-dependently, and completely inhibited gastric acid secretion at a concentration of 126 µM.

### [Example 3] Expression of somatostatin and CaSR in fractions of rat gastric mucosal D cells

The abdomen of each of male SD rats was opened in the midline under anesthesia to remove the stomach. The stomach was ligated at the cardiac region and at the junction between the gastric body and the pylorus, and the pyloric region was excised. After that, the stomach was everted so that the luminal side might be exposed outside. To the inside of the stomach, Buffer A containing 2.5 mg/ml protease (Sigma P6911, Protease, From *Streptomyces griseus,* 5. 5 unit/mg solid) was injected to prepare a balloon. The balloon was shaken at 37°C for 30 minutes in Buffer A for 30 minutes and in Buffer B for 30 minutes × three times. After that, the balloon was stirred three times in Buffer B containing 0.5 mg/ml DNase, the detached cells were centrifuged and then suspended in Buffer C. After the cell suspension had been filtered with a filter, cells were fractionated and collected with an elutriator manufactured by Hitachi, Ltd.

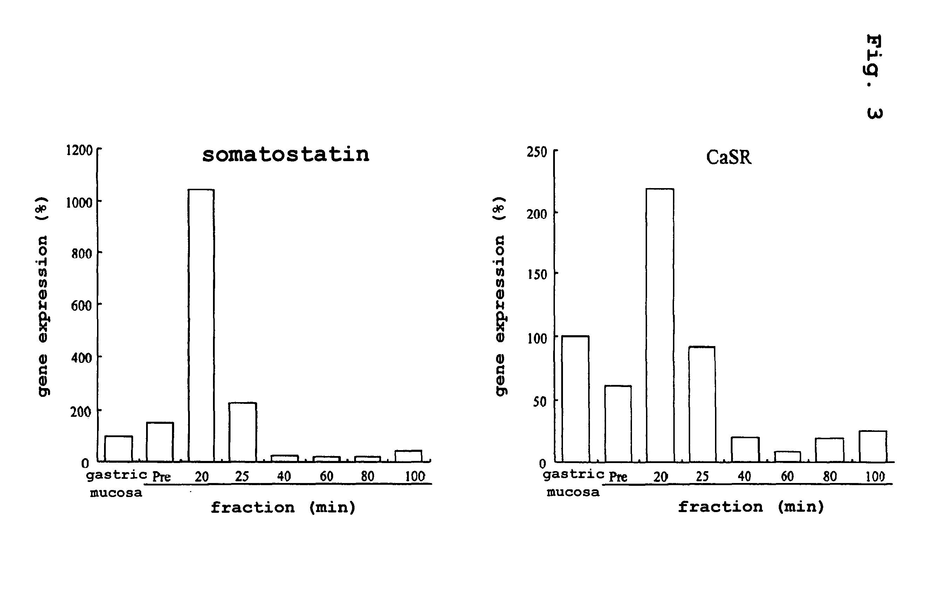

The mRNA expression amounts of somatostatin and CaSR in the above-described cell fractions and the entire gastric mucosa were measured by a real-time RT-PCR method. FIGS. 3 illustrate the mRNA expression amounts of somatostatin and CaSR in each of the above-described cell fractions in terms of relative values (gene expression (%)) to the mRNA expression amounts in the entire gastric mucosa. The results revealed that one part (20 ml/min) of the cell fractions highly expressed somatostatin and CaSR in an extremely specific manner as compared to the other parts of the cell fractions. Accordingly, it was considered that the cells fractionated under this condition were mainly D cells as somatostatin-producing cells, and the D cells expressed CaSR.

The composition of each of the buffers is as follows: Buffer A (70 mM NaCl, 5 mM KCl, 11 mM glucose, 50 mM HEPES, 0.5 mM NaH₂PO₄ 1.0 mM Na₂HPO₄, 20 mM NaHCO₃, 2 mM Na₂EDTA, 20 mg/ml BSA); Buffer B (70 mM NaCl, 5 mM KCl, 1.5 mM MgSO₄, 1.0 mM CaCl₂, 11 mM glucose, 50 mM HEPES, 0.5 mM NaH₂PO₄ 1.0 mM Na₂HPO₄, 20 mM NaHCO₃, 20 mg/ml BSA);
Buffer C (70 mM NaCl, 5 mM KCl, 1.5 mM MgSO₄, 1.0 mM CaCl₂, 11 mM glucose, 50 mM HEPES, 0.5 mM NaH₂PO₄, 1.0 mM Na₂HPO4, 20 mM NaHCO₃, 0.5 mM dithiothreitol, 1 mg/ml BSA).

### [Example 4] Somatostatin secretion in the case of treating fractionated D cells with CaSR agonist

Repeated fractionation was conducted using an elutriator in accordance with Example 3 to collect D cells with high purity. After centrifugation, the cells were suspended in a cell culture medium (D-MEM/F-12 containing 10% FBS) and cultured in a CO₂ incubator (95% O₂+5% CO₂, 37°C) (6×10⁵ cells/250 µL/well). After 2-day culture, to the D cells washed with PBS was added each of CaSR agonists (20 µM cinacalcet (CCT), 20 µM γ-EVG, 10 mM phenylalanine (Phe), and 10 mMhistidine (His)) mixed in a culture buffer (122 mM NaCl, 5 mM KCl, 25 mM NaHCO₃, 1.3 mM MgSO₄ 2 mM CaCl₂, 15 mM HEPES, and 20 mM glucose, pH 7.4), and the cells were cultured for 90 minutes. After that, a culture supernatant was collected, filtered with a filter, and then measured for its somatostatin concentration with a somatostatin ELISA kit (manufactured by Phoenix, California).

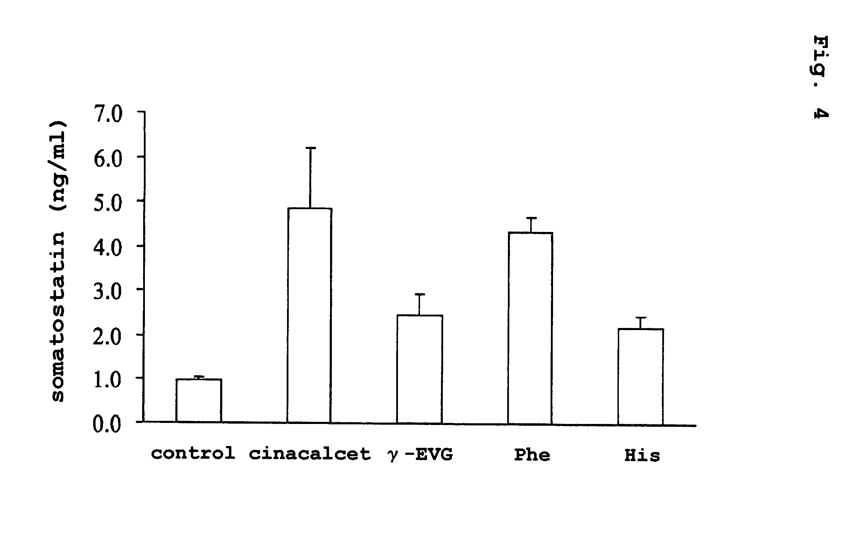

FIG. 4 illustrates the results. In the cultured D cells, cinacalcet, γ-EVG, Phe, and His each remarkably promoted somatostatin secretion.

### [Example 5] Gastric acid secretion in the case of using cinacalcet and somatostatin receptor 2 inhibitor in combination for histamine-induced gastric acid secretion

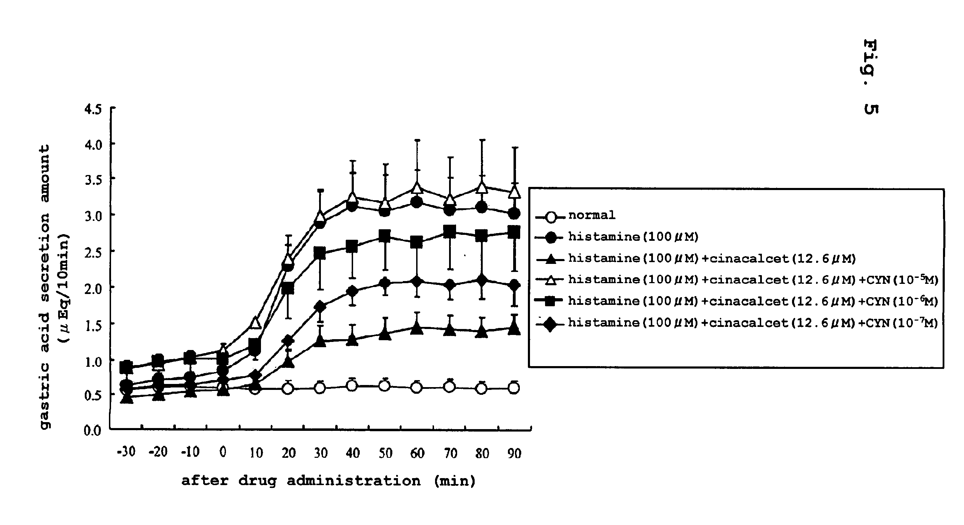

The gastric acid secretion was measured in accordance with the method of Example 2. FIG. 5 illustrates the results. 100 µM histamine promoted gastric acid secretion and the effect was inhibited by 12.6 µM cinacalcet. When the removed stomach left to stand still in an organ bath was treated with histamine, cinacalcet, and CYN154806 (SIGMA) (10⁻⁷ to 10⁻⁵ M) as an inhibitor selective to somatostatin receptor 2, the inhibition of gastric acid secretion with cinacalcet was released depending on the dose of CYN154806, resulting in the enhancement of gastric acid secretion. This suggested that cinacalcet affected somatostatin secretion to inhibit gastric acid secretion.

### [Example 6] Effect of cinacalcet or γ-EVG on non-steroidal anti-inflammatory drug (NSAID) -induced enteritis

To non-fasted rats, distilled water for injection containing cinacalcet (1, 3, 10, and 100 mg/kg) or γ-EVG (100 mg/kg), or distilled water for injection (control) was orally administered. After 30 minutes, loxoprofen (60 mg/kg) was orally administered, and the rats were left to stand for 24 hours. 1 ml of 1% (w/w) Evans Blue dye was intravenously administered, and after 30 minutes, the rats were killed under deep ether anesthesia. The small intestine (from the duodenum to the ileum) of each of the rats was removed, then immersed in 2% formalin for 10 minutes so that the small intestine might be fixed from the serosal side, and incised from the opposite side of the mesentery, and the damaged area in the small intestine (mm²) was measured with a dissecting microscope of 10 times power. The t-test or Dunnett's test was used for a statistical test and p<0.05 was considered significant.

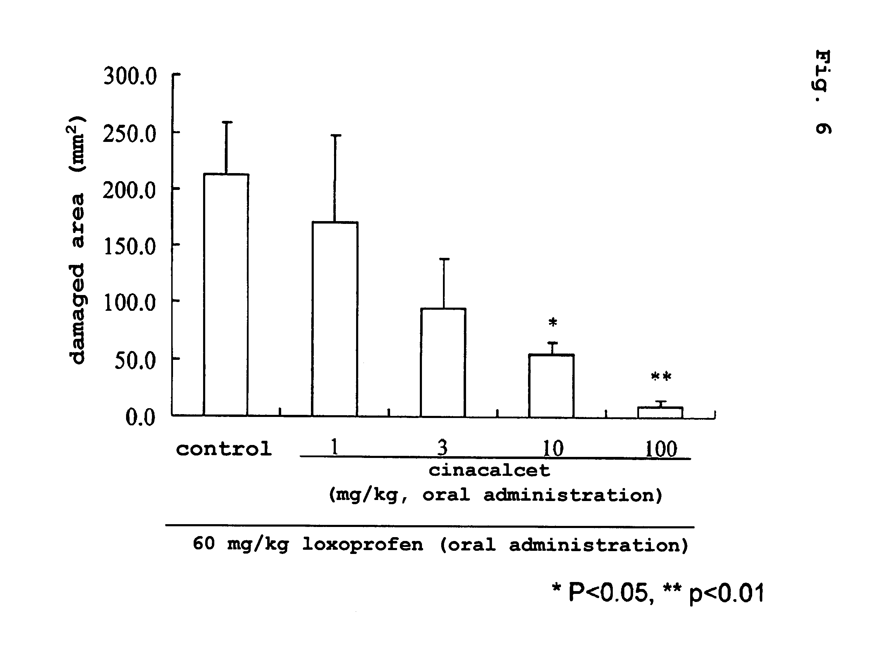

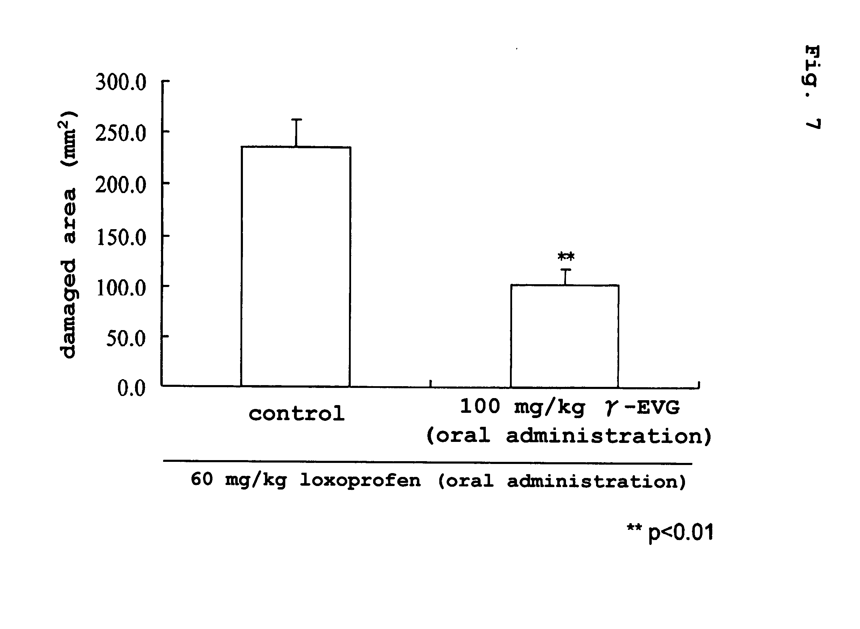

FIGS. 6 and 7 illustrate the results. Cinacalcet reduced the damaged area dose-dependently. Further, γ-EVG also reduced the damaged area. That is, the results showed that those medicaments as calcium receptor activators each had a prophylactic effect on NSAID-induced enteritis. It is understood that those medicaments are useful for the prophylaxis or treatment of NSAID-induced ulcer.

### [Example 7] Influence of γ-EVG on bicarbonate secretion in duodenum

The abdomen of each of male SD rats was opened under anesthesia, and a duodenum loop having a length of 1.7 cm from the pyloric ring of the stomach was prepared. The lumen of the loop was perfused with physiological saline saturated with 100% O₂ gas (flow rate: 1 ml/min).
After the bicarbonate secretion at the baseline had been measured for 30 minutes, distilled water for injection containing γ-EVG (100 mg/kg) or distilled water for injection (normal) was administered into the duodenal lumen, and the bicarbonate secretion amount was measured every 10 minutes.

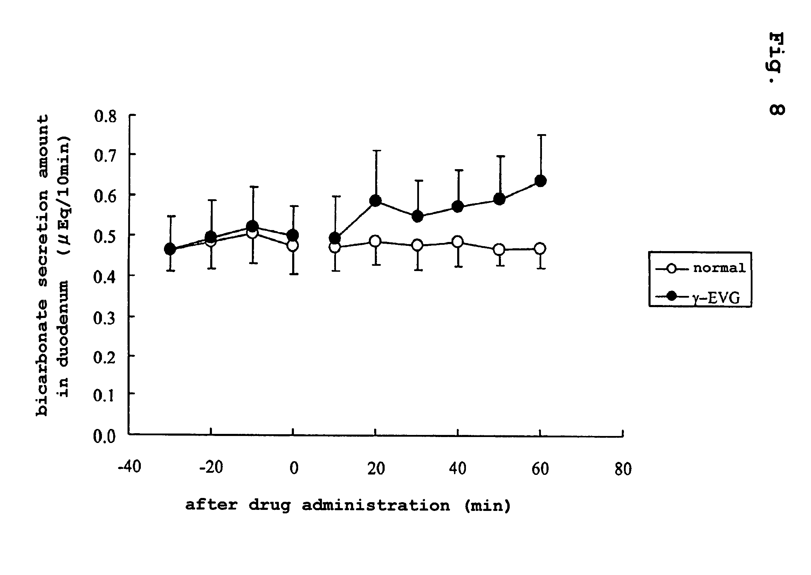

FIG. 8 illustrates the results. γ-EVG as a calcium receptor activator promoted bicarbonate secretion in the duodenum.

It is clear from the foregoing that the CaSR activator is effective for the promotion of bicarbonate secretion and for the treatment or prophylaxis of acid secretion-related diseases, for example.

### [Example 8] Somatostatin secretion in the case of treating fractionated gastric mucosal D cells with CaSR agonist and sodium glutamate

The somatostatin secretion was measured in accordance with the method of Example 4. After 2-day culture, to the D cells washed with PBS was added a CaSR agonist (20 µM γ-EVG), sodium glutamate (60 mM MSG), or a mixed solution thereof, mixed in a culture buffer (122 mM NaCl, 5 mM KCl, 25 mM NaHCO₃, 1.3 mM MgSO₄ 2 mM CaCl₂, 15 mM HEPES, and 20 mM glucose, pH7.4), and the cells was cultured for 90 minutes. After that, the culture supernatant was collected, filtered with a filter, and then the somatostatin concentration was measured with a somatostatin ELISA kit (Phoenix, California).

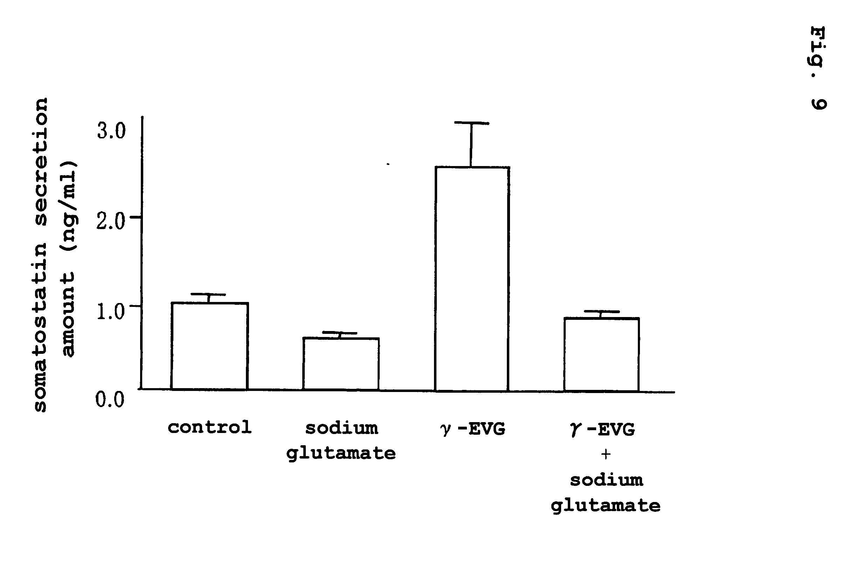

FIG. 9 illustrates the results. In the cultured D cells, γ-EVG markedly promoted somatostatin secretion. On the other hand, MSG remarkably inhibited somatostatin secretion. The mixed solution of γ-EVG and MSG inhibited somatostatin secretion.

### Industrial Applicability

The present invention provides a bicarbonate secretion promoter in the gastrointestinal tract. The bicarbonate secretion promoter in the gastrointestinal tract of the present invention can be utilized in a pharmaceutical or food for the prophylaxis or treatment or improvement of acid secretion-related diseases, particularly gastric ulcer, duodenal ulcer, NSAID-induced ulcer, GERD, NERD, and the like. The pharmaceutical or food of the present invention is excellent in safety.

## Claims

1. A bicarbonate secretion promoter in the gastrointestinal tract, comprising a calcium receptor activator as an active ingredient.

2. The bicarbonate secretion promoter according to claim 1, wherein the calcium receptor activator is a peptide.

3. The bicarbonate secretion promoter according to claim 1 or 2, wherein the peptide is selected from the group consisting of γ-Glu-X-Gly (X represents an amino acid or an amino acid derivative), γ-Glu-Val-Y (Y represents an amino acid or an amino acid derivative), γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, γ-Glu-Met(O), γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, and γ-Glu-Cys(S-Me).

4. The bicarbonate secretion promoter according to claim 3, wherein X is Cys, Cys(SNO), Cys(S-allyl), Gly, Cys(S-Me), Abu, or Ser, and Y is Gly, Val, Glu, Lys, Phe, Ser, Pro, Arg, Asp, Met, Thr, His, Orn, Asn, Cys, or Gln.

5. The bicarbonate secretion promoter according to claim 1, wherein the calcium receptor activator is selected from a compound represented by the following formula (1) or (2) and salts thereof.

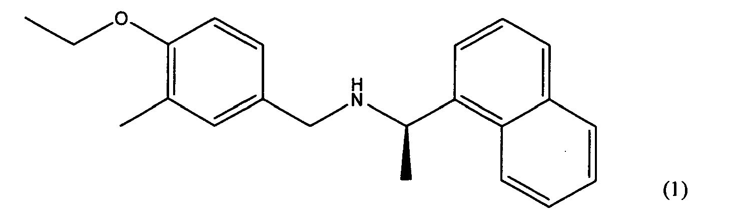

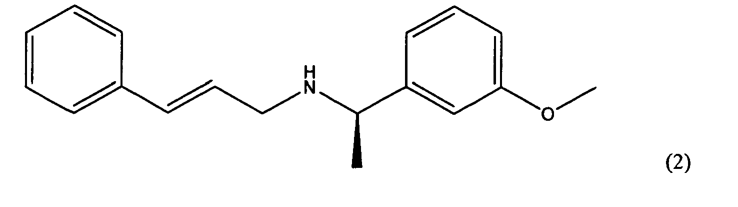

6. The bicarbonate secretion promoter according to claim 4, wherein the peptide is γ-Glu-Val-Gly.

7. The bicarbonate secretion promoter according to claim 1, wherein the calcium receptor activator is cinacalcet.

8. The bicarbonate secretion promoter according to any one of claims 1 to 7, which is a pharmaceutical used for the prophylaxis or treatment of an acid secretion-related disease.

9. The bicarbonate secretion promoter according to claim 8, wherein the acid secretion-related disease is selected from the group consisting of gastric ulcer, duodenal ulcer, non-steroidal anti-inflammatory drug-induced ulcer, gastro-esophageal reflux disease, and non-erosive reflux disease.

10. The bicarbonate secretion promoter according to claim 6 or 7, which is a pharmaceutical used for the prophylaxis or treatment of non-steroidal anti-inflammatory drug-induced ulcer.

11. The bicarbonate secretion promoter according to any one of claims 8 to 10, which is administered in an amount of 0.001 to 10 g/kg body weight per day for adults in terms of the amount of the calcium receptor activator.

12. A food comprising the bicarbonate secretion promoter according to any one of claims 1 to 11.

13. An appetite regulator comprising, as components, a somatostatin secretion promoter containing a calcium receptor activator as an active ingredient and an appetite stimulator, wherein one of the components is alternatively selected during use.

14. The appetite regulator according to claim 13, wherein the appetite stimulator is sodium L-glutamate.
